# EUROPÄISCHE PATENTSCHRIFT

(11) **EP 1 398 030 B1**
(45) Veröffentlichungstag und Bekanntmachung des Hinweises auf die Patenterteilung: **06.09.2006**
(21) Anmeldenummer: 03026947.6
(22) Anmeldetag: 28.05.1999
(51) Int. Cl.: A61K 31/425, A61K 31/40

(54) **Effektoren von Dipeptidylpeptidase IV**
Dipeptidyl peptidase IV effectors
Effecteurs de dipeptidylpeptidase IV

(30) Priorität: 28.05.1998 DE 19823831
(43) Veröffentlichungstag der Anmeldung: 17.03.2004
(62) Teilanmeldung aus: 03001324.7
(73) Patentinhaber: Prosidion Limited, Oxford, OX4 6LT (GB)
(72) Erfinder: Demuth, Hans-Ulrich, 06114 Halle (DE); Glund, Konrad, D-06120 Halle/Saale (DE); Schlenzig, Dagmar, 06114 Halle (DE); Kruber, Susanne, D-53177 Bonn (DE)
(74) Vertreter: Blakey, Alison Jane

(56) Entgegenhaltungen:
- EP-A- 0 837 069
- WO-A-97/40832
- WO-A-98/05331
- WO-A-98/19998
- DE-A- 4 140 177
- DE-A- 4 432 757
- US-A- 5 258 185

## Beschreibung

Die vorliegende Erfindung betrifft Dipeptidverbindungen bzw. zu Dipeptidverbindungen analoge Verbindungen, die aus einer Aminosäure und einer Thiazolidin- oder Pyrrolidingruppe gebildet werden, und deren Salze, im weiteren Dipeptidverbindungen genannt, und die Verwendung der Verbindungen zur Behandlung von beeinträchtigter Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern.

Die Erfindung betrifft also auch ein einfaches Verfahren zur Senkung der Blutzuckerkonzentration von Säugern mit Hilfe von Dipeptidverbindungen als aktivitätsmindernde Effektoren (Substraten, Pseudosubstraten, Inhibitoren, Bindungsproteinen, Antikörpern u. a.) für Enzyme mit vergleichbarer oder identischer Aktivität zur enzymatischen Aktivität des Enzyms Dipeptidyl Peptidase IV.

DP IV- bzw. DP IV-analoge Aktivität (z. B. besitzt die cytosolische DP II eine der DP IV nahezu identische Substratspezifität) kommt im Blutkreislauf vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen.

Die Glukose-abhängigen insulinotropen Polypeptide: Gastric Inhibitory Polypeptide 1-42 (GIP₁₋₄₂) und Glucagon-Like Peptide Amide-1 7-36 (GLP-l₇₋₃₆), also Hormone, die die Glukoseinduzierte Insulinsekretion des Pankreas stimulieren (auch Incretine genannt), sind Substrate der DP IV, da diese von den N-terminalen Sequenzen dieser Peptide die Dipeptide Tyrosinyl-Alanin bzw. Histidyl-Alanin in vitro und in vivo abspalten kann.

Die Reduktion derartiger DP IV- bzw. DP IV-analoger Enzymaktivität zur Spaltung solcher Substrate *in vivo* kann dazu dienen, unerwünschte Enzymaktivität unter Laborbedingungen wie auch bei pathologischen Zuständen von Säuger-Organismen wirksam zu unterdrücken. Z.B. basiert *Diabetes mellitus* Typ II (auch Altersdiabetes) auf einer verminderten Insulinsekretion bzw. Störungen in der Rezeptorfunktion, die u.a. in proteolytisch bedingten Konzentrationsanomalien der Incretine begründet sind.

Hyperglykämie und damit verbundene Ursachen bzw. Folgeerscheinungen (auch *Diabetes mellitus*) werden nach gegenwärtigem Stand der Technik durch die Verabreichung von Insulin (z.B. von aus Rinderpankreas isoliertem oder auch gentechnisch gewonnenem Material) an erkrankte Organismen in verschiedenen Darreichungsformen behandelt. Alle bisher bekannten, wie auch modernere Verfahren, zeichnen sich durch hohen Materialaufwand, hohe Kosten und oft durch entscheidende Beeinträchtigungen der Lebensqualität der Patienten aus. Die klassische Methode (tägliche *i.v.* Insulin-Injektion, üblich seit den dreißiger Jahren) behandelt die akuten Krankheitssymptome, führt aber nach längerer Anwendung u. a. zu schweren Gefäßveränderungen (Arteriosklerose) und Nervenschädigungen.

Neuerdings wird die Installation subkutaner Depot-Implantate (die Insulinabgabe erfolgt dosiert, und die täglichen Injektionen entfallen) sowie die Implantation (Transplantation) intakter Langerhansscher Zellen in die funktionsgestörte Pankreasdrüse oder andere Organe und Gewebe vorgeschlagen. Derartige Transplantationen sind technisch aufwendig. Weiterhin stellen sie einen risikobehafteten chirurgischen Eingriff in den Empfängerorganismus dar und verlangen auch bei Zellverpflanzungen nach Methoden zur Suppression bzw. der Umgehung des Immunsystems.

Die Verwendung von Alanyl-Pyrrolidid und Isoleucyl-Thiazolidid als Inhibitoren von DP IV bzw. von zu DP IV analoger Enzymaktivität ist bereits aus der PCT/DE 97/00820 und die Verwendung von Isoleucyl-Pyrrolidid und Isoleucyl-Thiazolidid-Hydrochlorid bereits aus der DD 296 075 bekannt. Bei dem in diesem Stand der Technik eingesetzten Isoleucyl-Thiazolidid handelt es sich um natürliches, also L-threo-Isoleucyl-Thiazolidid: Zum Prioritätsdatum und noch am Anmeldetag der beiden Druckschriften stand nur diese, die natürliche Form von Isoleucyl-Thiazolidid zur Verfügung.

Es ist festgestellt worden, daß diese Verbindungen, insbesondere L-threo-Isoleucyl-Thiazolidid, gute Effektoren für DP IV und DP IV analoge Enzymaktivitäten sind. Bei der Verwendung dieser Verbindung können bei einigen Patienten bzw. Krankheitsformen jedoch gewisse Probleme auftreten:
Je nach Symptomen und Schwere z.B. von Diabetes mellitus, wäre es z.B. wünschenswert, Effektoren zur Verfügung zu haben, die eine andere Wirkung als die bekannten Verbindungen aufweisen: So ist bekannt, daß Diabetes-mellitus-Patienten individuell "eingestellt" werden müssen, um eine optimale Behandlung ihrer Krankheit zu ermöglichen. So sollte bei einigen Fällen z.B. eine verringerte Aktivität von DP IV Effektoren ausreichen. Auch könnte eine zu hohe Inhibitor Aktivität und die permanente Verabreichung desselben Medikaments insbesondere wegen der lebenslangen Dauer der Behandlung unerwünschte Nebenwirkungen zur Folge haben. Weiter könnte es auch wünschenwert sein, gewisse Transporteigenschaften zur Erhöhung der Resorptionsgeschwindigkeit der Effektoren in vivo zu verbessern.

Aufgabe der Erfindung ist es daher, neue (insbesondere aktivitätsmindernde) Effektoren zur Behandlung von z.B. beeinträchtigter Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern und ein einfaches Verfahren zur Behandlung dieser Krankheiten bereitzustellen.

Diese Aufgabe wird erfindungsgemäß durch die Bereitstellung von Dipeptidverbindungen bzw. Analoga von Dipeptiden, die aus einer Aminosäure und einer Thiazolidin- oder Pyrrolidingruppe, wie in Anspruch 1 definiert, gebildet werden, und deren Salze gelöst.

Bei der - vorzugsweise oralen - Verabreichung dieser Effektoren an einen Säugerorganismus werden die endogenen (oder zusätzlich exogen verabreichten) insulinotropen Peptide GIP₁₋₄₂ und GLP-1₇₋₃₆ (o.a. GLP-1₇₋₃₇ oder deren Analoga) durch DP IV- oder DP IV-ähnliche Enzyme vermindert abgebaut und damit die Konzentrationsabnahme dieser Peptidhormone bzw. ihrer Analoga verringert bzw. verzögert. Der Erfindung liegt also der Befund zugrunde, daß eine Reduktion der im Blutkreislauf agierenden DP IV- oder DP IV-ähnlichen enzymatischen Aktivität zur Beeinflussung des Blutzuckerspiegels führt. Es wurde gefunden, daß
1. die Verminderung von DP IV- bzw. DP IV-analoger Aktivität zu relativer Stabilitätserhöhung der Glukosestimulierten, oder extern zugeführten Incretine (oder deren Analoga) führt, d.h. durch Applikation von Effektoren der DP IV bzw. DP IV-analoger Proteine der Incretin-Abbau im Blut kontrolliert werden kann;
2. die erhöhte biologische Abbaustabilität der Incretine (oder ihrer Analoga) eine Wirkungsveränderung endogenen Insulins zur Folge hat;
3. die durch Reduktion der DP IV- bzw. DP IV-analogen enzymatischen Aktivität im Blut erzielte Stabilitätserhöhung der Incretine in einer nachfolgenden Veränderung der Glukoseinduzierten Insulinwirkung resultiert und damit zu einer mittels DP IV-Effektoren kontrollierbaren Modulierung des BlutGlukosespiegels führt.

Dazu sind erfindungsgemäß Dipeptidverbindungen geeignet, bei denen die Aminosäure die natürliche Aminosäure Glutamin darstellt.

Die möglichst orale Applikation der erfindungsgemäßen hochaffinen, niedermolekularen Enzyminhibitoren ist eine kostengünstigere Alternative z.B. zu invasiven chirurgischen Techniken bei der Behandlung pathologischer Erscheinungen. Durch chemisches Design von Stabilitäts-, Transport- und Clearance-Eigenschaften kann deren Wirkungsweise modifiziert und auf individuelle Eigenschaften abgestimmt werden.

Wie vorstehend erwähnt, kann es z.B. bei der Dauerbehandlung von Diabetes mellitus erforderlich sein, Effektoren mit einer definierten Aktivität zur Verfügung zu stellen, mit denen individuelle Bedürfnisse von Patienten erfüllt bzw. Symptome behandelt werden können. Die erfindungsgemäßen Dipeptidverbindungen weisen daher bei einer Konzentration (der Dipeptidverbindungen) von 10 µM, insbesondere bei den in Tabelle 1 angegebenen Bedingungen, eine Aktivitätsminderung von Dipeptidyl Peptidase IV bzw. DP IV-analoger Enzymaktivitäten von mindestens 10, bevorzugt von mindestens 40 % auf. Häufig ist auch eine Aktivitätsminderung von mindestens 60 % bzw. mindestens 70 % erforderlich. Bevorzugte Effektoren können auch eine Aktivitätsminderung von maximal 20 % bzw. 30 % aufweisen. Weiterhin sind die Transporteigenschaften der vorliegenden Verbindungen, insbesondere durch den Peptidtransporter Pep T1 deutlich verbessert.

Die Salze der erfindungsgemäßen Dipeptidverbindungen können z.B. organische Salze wie Acetate, Succinate, Tartrate oder Fumarate oder anorganische Säurereste wie Phosphate oder Sulfate sein. Besonders bevorzugt werden die Fumarate, die eine hervorragende Wirkung bei einer überraschend hohen Stabilität gegenüber Hydrolyse aufweisen und wesentlich weniger löslich sind als die Hydrochloride. Diese Eigenschaften sind auch bei der Galenik von Vorteil.

Die Salze der Dipeptidverbindungen können in einem molaren Verhältnis von Dipeptid(analogon)komponente zu Salzkomponente von 1 : 1 oder 2 : 1 vorliegen.

Die Erfindung betrifft somit Effektoren der Dipeptidyl Peptidase IV (DP IV)- bzw. DP IV-analoger Enzymaktivität und deren Verwendung zur Senkung des Blutzuckerspiegels unter die für Hyperglykämie charakteristische Glukosekonzentration im Serum eines Säuger-Organismus. Insbesondere betrifft die Erfindung die Verwendung der erfindungsgemäßen Effektoren der DP IV- bzw. der DP IV-analogen Enzymaktivität zur Verhinderung oder Milderung pathologischer Stoffwechsel-Anomalien von Säuger-Organismen wie z.B. beeinträchtigte Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern. In einer weiteren bevorzugten Ausführungsform betrifft die Erfindung ein Verfahren zur Senkung des Blutzuckerspiegels unter die für Hyperglykämie charakteristische Glukosekonzentration im Serum eines Säuger-Organismus, das dadurch gekennzeichnet ist, daß man einem Säuger-Organismus eine therapeutisch wirksame Menge mindestens eines erfindungsgemäßen Effektors der DP IV- bzw. der DP IV-analogen Enzymaktivität verabreicht.

In einer weiteren bevorzugten Ausführungsform betrifft die Erfindung pharmazeutische Zusammensetzungen gemäß Anspruch 2, also Medikamente, die mindestens eine erfindungsgemäße Verbindung oder deren Salze in Kombination mit einem oder mehreren pharmazeutisch akzeptablen Trägern und/oder Lösungsmitteln enthalten.

Die pharmazeutischen Zusammensetzungen können z.B. als parenterale oder enterale Formulierungen vorliegen und entsprechende Träger enthalten bzw. sie können als orale Formulierungen vorliegen, die entsprechende zur oralen Verabreichung geeignete Träger enthalten können. Vorzugsweise liegen sie als orale Formulierungen vor.

Zusätzlich enthalten, die pharmazeutischen Zusammensetzungen einen oder mehrere hypoglykämisch wirkende Wirkstoffe, die an sich bekannte Wirkstoffe sein können.

Die erfindungsgemäßen Effektoren der DP IV bzw. der DP IV-analogen Enzymaktivität können zur Senkung des BlutzuckerSpiegels unter die für Hyperglykaemie charakteristische Glukose-Konzentration im Serum eines Säuger-Organismus bzw. zur Herstellung eines entsprechenden Medikaments verwendet werden.

Die erfindungsgemäß applizierten Effektoren der DP IV- bzw. DP IV-analoger Enzyme können in pharmazeutisch anwendbaren Formulierungen oder Formulierungskomplexen als Inhibitoren, Substrate, Pseudosubstrate, Inhibitoren der DP IV-Expression, Bindungsproteine oder Antikörper dieser Enzymproteine oder Kombinationen aus diesen verschiedenen Stoffen, die DP IV- bzw. DP IV-analoge Proteinkonzentration im Säugerorganismus reduzieren, zum Einsatz kommen. Erfindungsgemäße Effektoren sind Glutaminyl Pyrrolidin und Glutaminyl Thiazolidin (Tabelle 1) und deren Fumarsalze. Die erfindungsgemäßen Effektoren ermöglichen eine individuell einstellbare Behandlung von Patienten bzw. Krankheiten, wobei insbesondere individuell auftretende Unverträglichkeiten, Allergien und Nebenwirkungen vermieden werden können.

Auch weisen die Verbindungen unterschiedliche zeitliche Verläufe der Wirksamkeit auf. Dadurch wird dem behandelnden Arzt die Möglichkeit in die Hand gegeben, differenziert auf die individuelle Situation eines Patienten zu reagieren: Einerseits kann er die Geschwindigkeit des Eintritts der Wirkung und andererseits die Dauer der Wirkung und insbesondere die Stärke der Wirkung genau einstellen.

Das erfindungsgemäße Verfahren stellt eine neuartige Herangehensweise zur Senkung erhöhter Blutglukosekonzentration im Serum von Säugern dar. Es ist einfach, kommerziell nutzbar und zur Anwendung bei der Therapie, insbesondere von Erkrankungen, die auf überdurchschnittlichen Blutglukosewerten basieren, bei Säugern und insbesondere in der Humanmedizin geeignet.

Die Effektoren werden z.B. in Form von pharmazeutischen Präparaten verabreicht, die den Wirkstoff in Kombination mit üblichen aus dem Stand der Technik bekannten Trägermaterialien enthalten. Beispielsweise werden sie parenteral (z.B. i.v., in physiologischer Kochsalzlösung) oder enteral (z.B. oral, formuliert mit üblichen Trägermaterialien wie z. B. Glukose) appliziert.

In Abhängigkeit von ihrer endogenen Stabilität und ihrer Bioverfügbarkeit müssen pro Tag einfache oder auch mehrfache Gaben der Effektoren erfolgen, um die erwünschte Normalisierung der Blutglukosewerte zu erreichen. Z.B. kann ein solcher Dosisbereich beim Menschen im Bereich von 0.01 mg bis 30.0 mg pro Tag, vorzugsweise im Bereich von 0.01 bis 10 mg Effektorsubstanz pro Kilogramm Körpergewicht liegen.

Es wurde gefunden, daß durch Verabreichung von Effektoren der Dipeptidyl Peptidase IV bzw. DP IV-analoger Enzymaktivitäten im Blut eines Säugers, durch deren damit verbundene, temporäre Aktivitätsreduktion, in kausaler Folge die endogenen (oder zusätzlich exogen verabreichten) insulinotropen Peptide Gastric Inhibitory Polypeptide 1-42 (GIP₁₋₄₂) und Glucagon-Like Peptide Amide-1 7-36 (GLP-1₇₋₃₆) (o.a. GLP-1₇₋₃₇ oder deren Analoga) durch DP IV- und DP IV-ähnliche Enzyme vermindert abgebaut werden und damit die Konzentrationsabnahme dieser Peptidhormone bzw. ihrer Analoga verringert bzw. verzögert werden. Die durch die Wirkung von DP IV-Effektoren erzielte, erhöhte Stabilität der (endogen vorhandenen oder exogen zugeführten) Incretine oder ihrer Analoga, die damit vermehrt für die insulinotrope Stimulierung der Incretin-Rezeptoren der Langerhansschen Zellen im Pankreas zur Verfügung stehen, verändert u.a. die Wirksamkeit von körpereigenem Insulin, was eine Stimulierung des Kohlehydratstoffwechsels des behandelten Organismus nach sich zieht.

Als Resultat sinkt der Blutzuckerspiegel unter die für Hyperglykämie charakteristische Glukosekonzentration im Serum des behandelten Organismus. Damit können Stoffwechselanomalien wie beeinträchtigte Glukosetoleranz, Glukosurie, Hyperlipidämie sowie mögliche schwere metabolische Azidosen und Diabetes mellitus, Krankheitsbilder die Folge einer über einen längeren Zeitraum erhöhten Glukosekonzentrationen im Blut sind, verhindert bzw. gemildert werden.

In der Reihe der aus dem Stand der Technik bekannten, oral wirksamen Antidiabetika ist bisher eine derartig wirksame, niedermolekulare Substanzklasse (mit Ausnahme des Biguanides Metformin: Molekulargewicht 130) nicht bekannt. Die Molekulargewichte der Aminoacyl Thiazolidide bewegen sich zwischen 146 (Glycyl Thiazolidid), 203 (Isoleucyl Thiazolidid) und 275 (Tryptophanoyl Thiazolidid). Im Vergleich bewegen sich die Molekulargewichte der Sulphonylharnstoffe (Glibenclamid: 494), der Saccharide (Acarbose: 630) sowie der Thiazolidindione (Pioglitazon: 586) im Bereich um 500 bis 700 Da. Physiologisch werden Aminoacyl Thiazolidide durch Aminopeptidasen sowie durch saure Hydrolyse in körpereigene Substanzen, wie Aminosäuren und Cysteamin, hydrolysiert, so daß die Verwendung der erfindungsgemäßen Verbindungen als oral verfügbare Antidiabetika eine Bereicherung der Pharmazie darstellt.

Bei Ratten und Mäusen ist experimentell induzierte Hyperglykämie durch orale Verabreichung mit den erfindungsgemäß verwendeten Verbindungen überdurchschnittlich gut behandelbar (Tabellen 2 und 3). Die Verabreichung des 500- bis 1000-fachen der wirksamen Dosis führte zu keiner nachweisbaren pathologischen Veränderung während drei-wöchiger toxikologischer Experimente an Ratten und Mäusen.

Die vorteilhafte Wirkung von erfindungsgemäßen Verbindungen auf DP IV ist in Tabelle 1 beispielhaft belegt:

**Tabelle 1: Wirkung verschiedener Effektoren auf die durch Dipeptidyl Peptidase IV katalysierte Hydrolyse von 0,4 mM des Substrates H-Gly-Pro-pNA bei 30°C, pH 7,6 und einer Ionenstärke von 0,125.**

| **Effektor** | **Effektoraffinität zu DP IV: K**_{**i**} **[nM]** | **% Restaktivität der DP IV in Gegenwart von 10 µM Effektor** |
|---|---|---|
| Metformin | » 1.000.000 | 100 |
| Glibenclamid | » 1.000.000 | 100 |
| Acarbose | » 1.000.000 | 100 |
| H-Asn-Pyrrolidid | 12.000 | 83,1 |
| H-Asn-Thiazolidid | 3.500 | 47,2 |
| H-Asp-Pyrrolidid | 14.000 | 81,6 |
| H-Asp-Thiazolidid | 2.900 | 45,6 |
| H-Asp(NHOH)-Pyrrolidid | 13.000 | 88,2 |
| H-Asp(NHOH)-Thiazolidid | 8.800 | 54,5 |
| H-Glu-Pyrrolidid | 2.200 | 38,5 |
| H-Glu-Thiazolidid | 610 | 25,0 |
| H-Glu(NHOH)-Pyrrolidid | 2.800 | 44,9 |
| H-Glu(NHOH)-Thiazolidid | 1.700 | 36,5 |
| H-His-Pyrrolidid | 3.500 | 49,7 |
| H-His-Thiazolidid | 1.800 | 35,2 |
| H-Pro-Pvrrolidid | 4.100 | 50,2 |
| H-Pro-Thiazolidid | 1.200 | 27,2 |
| H-Ile-Azididid | 3.100 | 43,8 |
| H-Ile-Pyrrolidid | 210 | 12,3 |
| **H-L-allo-Ile-Thiazolidid** | **190** | **10,0** |
| H-Val-Pyrrolidid | 480 | 23,3 |
| H-Val-Thiazolidid | 270 | 13,6 |

Es ist bekannt, daß Aminoacyl Pyrrolidide und Aminoacyl Thiazolidide durch die in den Mucosazellen des Dünndarms, im Serum und in Leberzellen vorhandenen Enzyme Prolin Aminopeptidase und Prolidase abgebaut werden können und der Thiazolidinring zur Öffnung in Gegenwart von Säuren (beispielsweise im Magen) unter Bildung des adäquaten Cysteamin-Derivates neigt [vgl. US 458407]. Es war daher überraschend, eine dosisabhängige Wirksamkeit der Wirkstoffe nach per oraler Verabreichung zu finden. Die Dosisabhängigkeit der Wirkung von L-allo-Ile-Thiazolidid auf die Serum-DP IV Aktivität nach oraler Applikation von L-allo-Isoleucyl Thiazolidid an gesunden Wistarratten ist mit folgender Tabelle belegt

**Tabelle 2: Restaktivität der DP IV im Serum gegenüber 0,4 mM des Substrates H-Gly-Pro-pNA bei 30°C, pH 7,6 und einer Ionenstärke von 0,125, nach oraler Gabe und in Abhängigkeit von der Dosis L-allo-Isoleucyl Thiazolidid, bestimmt 30 min nach Applikation des Inhibitors.**

| Dosis pro Versuchstier | Restaktivität der DP IV in % |
|---|---|
| 0 mg | 100 |
| 2,5 mg | 52 |
| 5,0 mg | 40 |
| 10 mg | 28 |
| 20 mg | 29 |

Ausgesprochen überraschend ist die im diabetischen Tiermodell erzielte Glukose-reduzierende Wirkung des Wirkstoffs L-allo-Isoleucyl Thiazolidid nach seiner oralen Verabreichung bei zeitgleicher oraler Glukose-Stimulierung (Tabelle 3).

Zur Verstärkung der blutzuckersenkenden Wirkung verschiedener Antidiabetika werden häufig Kombinationen verschiedener oral wirksamer Antidiabetika eingesetzt. Da sich die äntihyperglykämische Wirkung der erfindungsgemäßen Effektoren unabhängig von anderen bekannten oral applizierbaren Antidiabetika entfaltet, eignen sich die erfindungsgemäßen Wirkstoffe analog in entsprechender galenischer Form, zur Erzielung des gewünschten normoglykämischen Effektes, zum Einsatz bei Kombinationstherapien.

Somit können die erfindungsgemäß verwendeten Verbindungen in an sich bekannter Weise in die üblichen Formulierungen überführt werden, wie z. B. Tabletten, Kapseln, Dragees, Pillen, Suppositorien, Granulate, Aerosole, Sirupe, flüssige, feste und cremeartige Emulsionen und Suspensionen und Lösungen unter Verwendung inerter, untoxischer, pharmazeutisch geeigneter Träger- und Zusatzstoffe oder Lösungsmittel. Hierbei liegen die therapeutisch wirksamen Verbindungen jeweils vorzugsweise in einer Konzentration von etwa 0.1 bis 80, vorzugsweise von 1 bis 50 Masseprozent der Gesamtmischung vor, d.h. in Mengen, die ausreichend sind, um den angegebenen Dosierungsspielraum zu erreichen.

**Tabelle 3: Reduktion der zirkulierenden Blutglukose innerhalb 60 min nach oraler Verabreichung von 20 µM L-allo-Ile-Thiazolidid an Ratten verschiedener Tiermodelle bei zeitgleichem Glukosetoleranztest (Angaben in % bezogen auf normoglykämische Werte).**

| Tiermodell | Glukosekonzentration in % Kontrolle | Glukosekonzentration in % L-allo-Ile-Thiazolidid behandelt |
|---|---|---|
| Wistarratte, normal | 100 | 82 |
| Wistarratte (Diabetes 2b - Model, dick) | 100 | 73 |

Die gute Resorption der erfindungsgemäß verwendeten Verbindungen durch Schleimhäute des gastrointestinalen Traktes ermöglicht die Anwendung von vielen galenischen zubereitungen:
Die Substanzen können als Medikament in Form von Dragees, Kapseln, Beißkapseln, Tabletten, Tropfen, Sirup, aber auch als Zäpfchen oder als Nasensprays angewendet werden.

Die Formulierungen werden beispielsweise hergestellt durch Strecken des Wirkstoffs mit Lösungsmitteln und/oder Trägerstoffen, gegebenenfalls unter Verwendung von Emulgatoren und/oder Dispergiermitteln, wobei z. B. im Fall des Einsatzes von Wasser als Verdünnungsmittel gegebenenfalls organische Lösungsmittel als Hilfslösungsmittel verwendet werden können.

Als Hilfsstoffe seien beispielhaft aufgeführt: Wasser, untoxische organische Lösungsmittel, wie Paraffine (z. B. Erdölfraktionen), pflanzliche Öle (z. B. Rapsöl, Erdnußöl, Sesamöl), Alkohole (z.B. Ethylalkohol, Glycerin), Glykole (z.B. Propylenglykol, Polyetylenglykol); feste Trägerstoffe, wie z. B. natürliche Gesteinsmehle (z. B. hochdisperse Kieselsäure, Silikate), Zucker (z.B. Roh-, Milch- und Traubenzucker); Emulgiermittel, wie nichtionogene und anionische Emulgatoren (z. B. Polyoxyethylen-Fettsäure-Ester, Polyoxyethylen-FettalkoholEther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) und gegebenenfalls Aromastoffe.

Die Applikation erfolgt in üblicher Weise, vorzugsweise enteral oder parenteral, insbesondere oral. Im Falle der enteralen Anwendung können Tabletten außer den genannten Trägerstoffen weitere Zusätze wie Natriumcitrat, Calciumcarbonat und Calciumphosphat, zusammen mit verschiedenen Zuschlagsstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den oben genannten Hilfsstoffen zusätzlich mit verschiedenen Geschmacksaufbesserern oder Farbstoffen versetzt werden.

Bei einer parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von etwa 0,01 bis 2,0 mg/kg, vorzugsweise etwa 0,01 bis 1,0 mg/kg Körpergewicht pro Tag zur Erreichung wirksamer Ergebnisse zu verabreichen, und bei enteraler Applikation beträgt die Dosierung etwa 0,01 bis 2 mg/kg, vorzugsweise etwa 0,01 bis 1 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres oder Patienten bzw. der Art des Applikationsweges, aber auch aufgrund der Tierart und deren individuellem verhalten gegenüber dem Medikament bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

### Beispiele für pharmazeutische Formulierungen

### 1. Kapseln mit 100 mg L-allo-Isoleucyl Thiazolidid pro Kapsel (Referenz):

Für ca. 10 000 Kapseln wird eine Lösung folgender zusammensetzung hergestellt:

| | |
|---|---|
| L-allo-Isoleucyl Thiazolidid Hydrochlorid | 1,0 kg |
| Glycerin | 0,5 kg |
| Polyethylenglykol | 3,0 kg |
| Wasser | 0,5 kg |
| | 5,0 kg |

Die Lösung wird nach an sich bekannter Weise in Weichgelatinekapseln abgefüllt. Die Kapseln sind zum Zerbeißen oder zum Schlucken geeignet.

### 2. Tabletten bzw. lackierte Tabletten oder Dragees mit 100 mg L-allo-Isoleucyl Thiazolidid (Referenz):

Die folgenden Mengen beziehen sich auf Herstellung von 100 000 Tabletten:

| | |
|---|---|
| L-allo-Isoleucyl Thiazolidid Hydrochlorid, fein vermahlen | 10,0 kg |
| Glukose | 4,35 kg |
| Milchzucker | 4,35 kg |
| Stärke | 4,50 kg |
| Zellulose, fein vermahlen | 4,50 kg |

Obige Bestandteile werden gemischt und anschließend mit einer Lösung, hergestellt aus

| | |
|---|---|
| Polyvinylpyrrolidon | 2,0 kg |
| Polysorbat | 0,1 kg |
| und Wasser | ca. 5,0 kg |

versehen und in an sich bekannter Weise granuliert, indem die feuchte Masse geraspelt und nach Zugabe von 0,2 kg Magnesiumstearat getrocknet wird. Die fertige Tablettenmischung von 30,0 kg wird zu gewölbten Tabletten von 300 mg Gewicht verarbeitet. Die Tabletten können nach an sich bekannter Weise lackiert oder dragiert werden.

Die technischen Daten bevorzugter Verbindungen werden nachstehend angegeben.

### Untersuchungen zu Ile-Thia*Fumarat (Isomere) und anderen Salzen

| **Substanz** | **K**_{**i**} | **Fp (°C)** | **CE (min)** | **MS** | **[α]H**_{**2**}**O** |
|---|---|---|---|---|---|
| L-threo-IT* F | 8*10⁻⁸ | 150^{DSC} | 160 | 203 | -10,7 (405nm) |
| D-threo-IT*F | keine Hemmung | 147 | 158 | 203 | nicht bestimmt |
| L-allo-IT*F | 2*10⁻⁷ | 145-6 | 154 | 203 | -4,58 (380nm) |
| D-allo-IT*F | keine Hemmung | 144-6 | 150 | 203 | 4,5 (380nm) |

| | | | | | |
|---|---|---|---|---|---|
| IT*F = Isoleucyl-Thiazolidid Fumarat Die NMR und HPLC Daten bestätigen, daß es sich um die entsprechenden Substanzen handelte | | | | | |

**Meßbedingungen für die Kᵢ-Bestimmung der Substanzen**

| | |
|---|---|
| Enzym: | DPIV_{Schweineniere}, 0,75mg/ml, 18U/ml (GPpNA) in 25mM Tris pH 7,6,30% Ammoniumsulfat, 0,5mM EDTA, 0,5mM DTE *Stammlösung:* 1:250 verdünnt in Meßpuffer |
| Puffer: | 40mM HEPES pH 7,6,1=0,125 (KCl) |
| Substrat: | GPpNA*HCl *Stammlösung:* 2,1mM |
| Meßgerät: | Perkin-Elmer Bio Assay Reader, HTS 7000 Plus, T=30°C λ= 405nm |
| Meßansatz: | 100µl Puffer 100µl Substrat (3 verschiedene Konzentrationen 0,8mM - 0,2mM) 50µl Wasser/Inhibitor (7 verschiedene Konzentrationen 2,1µM - 32,8nM) 10µl Enzym |

Puffer, Wasser/Inhibitor und Enzym wurden auf 30°C vortemperiert und die Reaktion durch die Zugabe von ebenfalls vortemperiertem Substrat gestartet.
Es wurden 4fach Bestimmungen durchgeführt.
Die Meßzeit betrug 10min.

### Schmelzpunktbestimmung

Schmelzpunkte wurden an einem Kofler-Heiztischmikroskop der Leica-Aktiengesellschaft, die Werte sind nicht korrigiert, oder an einem DSC-Gerät (bei Heumann-Pharma) bestimmt.

### Optische Rotation

Die Drehwerte wurden bei unterschiedlichen Wellenlängen an einem "Polarimeter 341" oder höher der Fa. Perkin Elmer aufgenommen.

### Meßbedingungen für die Massenspektrometrie

Die Massenspektren wurden an einem "API 165" bzw. "API 365" der Fa. PE Sciex mittels Elektrosprayionisation (ESI) aufgenommen.
Es wird mit einer ungefähren Konzentration von c = 10 µg/ml gearbeitet, die Substanz wird in MeOH / H₂O 50:50, 0,1 % HCO₂H aufgenommen, die Infusion erfolgt mit Spritzenpumpe (20µl/min).Die Messungen erfolgten im Positivmodus [M+H]⁺, die ESI-Spannung beträgt U = 5600V.

Die Salze weisen die folgenden Daten auf:

| **IT*Salz** | **K**_{**i**} | **M (gmol**^{**-1**}**)** | **Fp (°C)** |
|---|---|---|---|
| Succinat | 5,1 e-8 | 522,73 | 116 |
| Tartrat | 8,3 e-8 | 352,41 | 122 |
| Fumarat | 8,3 e-8 | 520,71 | 156 |
| Hydrochlorid | 7,2 e-8 | 238,77 | 169 |
| Phosphat | 1,3 e-7 | 300,32 | 105 |

### Löslichkeitsuntersuchung der Salze des Ile-Thia

Ile-Thia*Fum
   Einwaage 10,55 mg
   entspricht 0,02 mmol (520,72 g/mol)
   Zugabe von 100 µl H₂O_{dest}
   100 µl keine Lösung, optisch: keine Oberflächenbenetzung
   ab 200 µl sukzessiver Beginn der Löslichkeit
   bei 400µl ist eine vollständiges Lösen zu beobachten 2,63 %
   Bei diesem Salz wurde also festgestellt, daß es kaum benetzbar ist und sich nicht zersetzt.
Ile-Thia*Succ
   Einwaage 16,6 mg
   entspricht 0,031 mmol (522,73 g/mol)
   Zugabe von 16 µl H₂O_{dest}
   16 µl keine Lösung, optisch "Aufsaugen" der Feuchtigkeit
   von 66 µl - 1,5 ml kein vollständiges Lösen der Substanz zu beobachten
Ile-Thia*Tartrat
   Einwaage 17,3 mg
   entspricht 0,049 mmol (352,41 g/mol)
   Zugabe von 100 µl H₂O_{dest}
   100 µl vollständiges Lösen
   17,3 %
Ile-Thia*Phos
   Einwaage 15,5 mg
   entspricht 0,051 mmol (300,32 g/mol)
   Zugabe von 100 µl H₂O_{dest}
   100 µl Anlösen ist zu beobachten
   sukzessive Zugabe von 100 µl H₂O
   bei 400 µl vollständiges Lösen
   3,87 %
Ile-Thia*HCl
   Einwaage 16,1 mg
   entspricht 0,067 mmol (238,77 g/mol)
   Zugabe von 100 µl H₂O_{dest}
   bei 100 µl vollständiges Lösen
   16,1 %

### Allgemeine Synthese Ile-Thia*Salz

Die Boc-geschüzte Aminosäure Boc-Ile-OH wird in Essigsäureethylester vorgelegt, der Ansatz wird auf ca. -5 °C gekühlt. N-Methylmorpholin wird zugetropft, Pivalinsäurechlorid (Labor) bzw. Neohexanoylchlorid (Technikum) wird unter Temperaturkonstanz zugetropft. Die Reaktion wird für wenige Minuten zur Aktivierung gerührt. N-Methylmorpholin (Labor) und Thiazolidinhydrochlorid (Labor) werde nacheinander zugetropft, Thiazolidin (Technikum) wird zugegeben. Die Aufarbeitung im Labor erfolgt klassisch mit Salzlösungen, im Technikum wird der Ansatz mit NaOH- und CH₃COOH-Lösungen gereinigt.
Die Abspaltung der Boc-Schutzgruppe wird durch HCl / Dioxan (Labor) bzw. H₂SO₄ (Technikum) erzielt.
Im Labor wird das Hydrochlorid aus EtOH / Ether kristallisiert.
Im Technikum wird das freie Amin durch Zugabe von NaOH / NH₃ dargestellt. Fumarsäure wird in heißen Ethanol gelöst, das freie Amin wird zugetropft, es fällt (Ile-Thia)₂-Fumarat (M = 520,71 gmol⁻¹).

Die Analytik von Isomeren bzw. Enantiomeren erfolgt durch Elektrophorese.

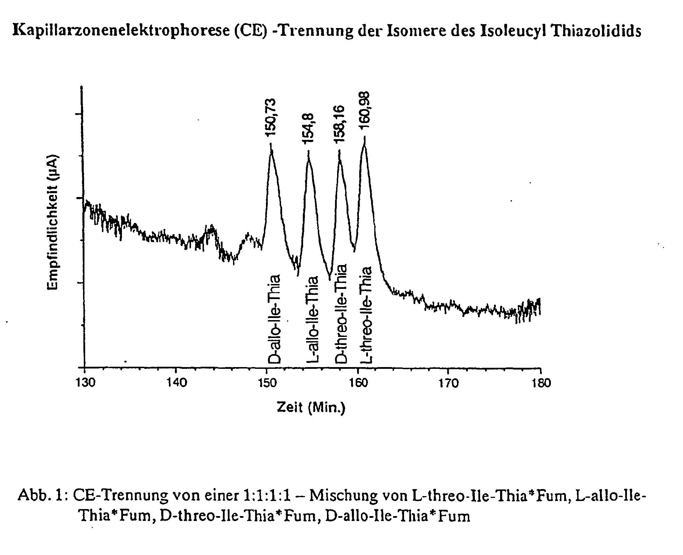

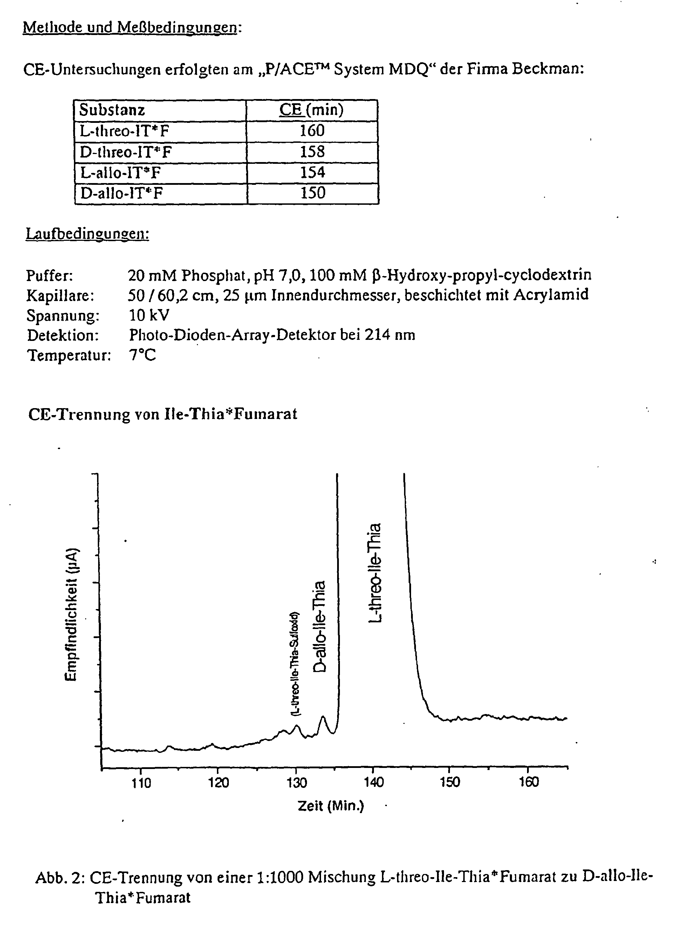

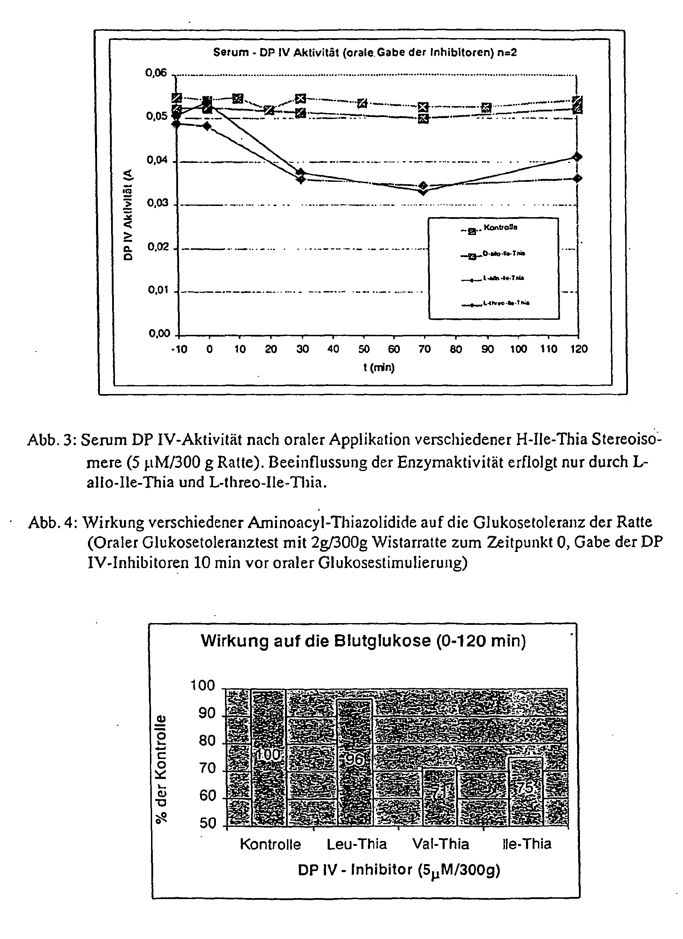

## Patentansprüche

1. Zusammensetzung, umfassend einen Effektor der Dipeptidyl Peptidase IV oder Dipeptidyl Peptidase IV-analoger Enzymaktivität und ein weiteres Antidiabetikum, worin der Effektor Glutaminyl Pyrrolidin, Glutaminyl Thiazolidin oder deren Salze ist.

2. Pharmazeutische Zusammensetzung, umfassend einen Effektor der Dipeptidyl Peptidase IV oder Dipeptidyl Peptidase IV-analoger Enzymaktivität und ein weiteres Antidiabetikum, und einen oder mehrere pharmazeutisch akzeptable Träger und/oder Lösungsmittel, worin der Effektor Glutaminyl Pyrrolidin, Glutaminyl Thiazolidin oder deren Salze ist.

3. Verwendung von Glutaminyl Pyrrolidin, Glutaminyl Thiazolidin oder deren Salzen zur Herstellung eines Medikaments für den Gebrauch in Verbindung mit einem weiteren Antidiabetikum zur Behandlung von Stoffwechselerkrankungen in Säugern.

4. Verwendung einer Zusammensetzung oder einer pharmazeutischen Zusammensetzung nach einem der vorstehenden Ansprüche zur Herstellung eines Medikaments zur Behandlung von Stoffwechselerkrankungen in Säugern.

5. Die Verwendung nach Anspruch 3 oder 4, **dadurch gekennzeichnet, dass** die Stoffwechselerkrankung aus beeinträchtigter Glukosetoleranz, Glukosurie, Hyperlipidämie, metabolischen Azidosen, Diabetes mellitus, diabetischer Nephropathie und Neuropathie sowie aus durch Diabetes mellitus verursachten Folgeerkrankungen ausgewählt ist.

6. Die Verwendung nach einem der Ansprüche 3 bis 5 zur Erhöhung der Stabilität von mindestens einem, entweder endogen vorhandenen oder exogen verabreichten, GIP₁₋₄₂, GLP-1₇₋₃₆ oder GLP-1₇₋₃₇ oder deren Analoga.

7. Die Verwendung nach einem der Ansprüche 3 bis 6 zur Erhöhung der Incretinspiegel und zur Stimulierung der Incretinrezeptoren der Langerhans'schen Zellen.

8. Die Verwendung nach einem der Ansprüche 3 bis 7 zur Erhöhung der Effektivität der Langerhans'schen Zellen.

9. Die Verwendung nach einem der Ansprüche 3 bis 8 zur Stimulierung des Kohlehydratstoffwechsels in Säugern.

10. Die Verwendung nach einem der Ansprüche 3 bis 9 zur Senkung des Blutglukosespiegels im Serum eines Säugers unter die für Hyperglykämie charakteristische Glukosekonzentration.

11. Die Verwendung nach einem der Ansprüche 3 bis 10, worin das Medikament zur oralen Behandlung von Säugern ist.

## Claims

1. Composition comprising an effector of dipeptidyl pepidase IV or dipeptidyl peptidase IV-analogous enzyme activity and a further antidiabetic, wherein the effector is glutaminyl pyrrolidine, glutaminyl thiazolidine or their salts.

2. Pharmaceutical composition comprising an effector of dipeptidyl peptidase IV or dipeptidyl peptidase IV-analogous enzyme activity and a further antidiabetic, and one or more pharmaceutically acceptable excipients and/or solvents, wherein the effector is glutaminyl pyrrolidine, glutaminyl thiazolidine or their salts.

3. Use of glutaminyl pyrrolidine, glutaminyl thiazolidine or their salts for producing a medicament for use in conjunction with a further antidiabetic for treating metabolic disaeses in mammals.

4. Use of a composition or of a pharmaceutical composition according to one of the preceding claims for producing a medicament for treating metabolic diseases in mammals.

5. Use according to Claim 3 or 4, **characterized in that** the metabolic disease is selected from impaired glucose tolerance, glucosuria, hyperlipidaemia, metabolic acidosis, diabetes mellitus, diabetic nephropathy and neuropathy and sequelae caused by diabetes mellitus.

6. Use according to one of Claims 3 to 5 for increasing the stability of at least one either endogeneously present or exogenously administered GIP₁₋₄₂, GLP-1₇₋₃₆ or GLP-1₇₋₃₇ or their analogues.

7. Use according to one of Claims 3 to 6 for increasing the incretin level and for stimulating the incretin receptors of Langerhans cells.

8. Use according to one of Claims 3 to 7 for increasing the efficiency of Langerhans cells.

9. Use according to one of Claims 3 to 8 for stimulating carbohydrate metabolism in mammals.

10. Use according to one of Claims 3 to 9 for lowering the blood sugar level in the serum of a mammal below the glucose concentration which is characteristic of hyperglycaemia.

11. Use according to one of Claims 3 to 10, in which the medicament is for orally treating mammals.

## Revendications

1. Composition comprenant un effecteur de la dipeptidylpeptidase IV ou d'une activité enzymatique analogue à la dipeptidylpeptidase IV et un agent antidiabétique supplémentaire, dans laquelle l'effecteur est la glutaminylpyrrolidine, la glutaminylthiazolidine ou leurs sels.

2. Composition pharmaceutique comprenant un effecteur de la dipeptidylpeptidase IV ou d'une activité enzymatique analogue à la dipeptidylpeptidase IV et un agent antidiabétique supplémentaire, et un ou plusieurs supports et/ou solvants pharmaceutiquement acceptables, dans laquelle l'effecteur est la glutaminylpyrrolidine, la glutaminylthiazolidine ou leurs sels.

3. Utilisation de la glutaminylpyrrolidine, de la glutaminylthiazolidine ou de leurs sels pour la fabrication d'un médicament destiné à être utilisé en association avec un agent antidiabétique supplémentaire pour le traitement de maladies métaboliques chez des mammifères.

4. Utilisation d'une composition ou d'une composition pharmaceutique selon l'une quelconque des revendications précédentes, pour la fabrication d'un médicament destiné au traitement de maladies métaboliques chez les mammifères.

5. Utilisation selon la revendication 3 ou 4, **caractérisée en ce que** la maladie métabolique est choisie parmi la tolérance altérée au glucose, la glycosurie, l'hyperlipidémie, des acidoses métaboliques, le diabète sucré, une néphropathie et neuropathie diabétique, ainsi que des maladies secondaires provoquées par le diabète sucré.

6. Utilisation selon l'une quelconque des revendications 3 à 5 pour accroître la stabilité d'au moins un GIP₁₋₄₂, GLP-1₇₋₃₆ ou GLP-1₇₋₃₇, soit présent par voie endogène, soit administré par voie exogène, ou de leurs analogues.

7. Utilisation selon l'une quelconque des revendications 3 à 6 pour accroître le taux d'incrétine et pour stimuler les récepteurs de l'incrétine ou les cellules de Langerhans.

8. Utilisation selon l'une quelconque des revendications 3 à 7 pour accroître l'efficacité des cellules de Langerhans.

9. Utilisation selon l'une quelconque des revendications 3 à 8 pour stimuler le métabolisme des hydrates de carbone.

10. Utilisation selon l'une quelconque des revendications 3 à 9 pour abaisser le taux de glycémie dans le sérum d'un mammifère en dessous de la concentration en glucose caractéristique de l'hyperglycémie.

11. Utilisation selon l'une quelconque des revendications 3 à 10, dans laquelle le médicament est destiné au traitement par voie orale de mammifères.
